(19) Europäisches Patentamt
European Patent Office
Office européen des brevets

(11) **EP 3 659 648 B1**

(12) **EUROPEAN PATENT SPECIFICATION**

(45) Date of publication and mention
of the grant of the patent:
**23.08.2023 Bulletin 2023/34**

(21) Application number: **18838081.0**

(22) Date of filing: **18.07.2018**

(51) International Patent Classification (IPC):
***A61M 5/315*** *(2006.01)*      ***A61M 5/31*** *(2006.01)*

(52) Cooperative Patent Classification (CPC):
**A61M 5/3129; A61M 5/31513;** A61M 2005/3104;
A61M 2005/3131

(86) International application number:
**PCT/JP2018/026900**

(87) International publication number:
**WO 2019/021906 (31.01.2019 Gazette 2019/05)**

(54) **SYRINGE BARREL AND SYRINGE**

SPRITZENZYLINDER UND SPRITZE

CYLINDRE DE SERINGUE ET SERINGUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.07.2017 JP 2017142873**

(43) Date of publication of application:
**03.06.2020 Bulletin 2020/23**

(73) Proprietor: **Mitsubishi Gas Chemical Company,
Inc.**
**Tokyo 100-8324 (JP)**

(72) Inventors:
• **MINEZAKI, Takuya**
**Hiratsuka-shi**
**Kanagawa 254-0016 (JP)**

• **ITO, Fumihiro**
**Tokyo 100-8324 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 796 159**      **EP-A1- 2 957 310**
**WO-A1-2011/143509**      **WO-A1-2013/071138**
**JP-A- H10 314 305**      **JP-A- 2006 045 528**
**JP-A- 2016 019 619**      **US-A1- 2016 017 490**

**Description**

Technical Field

The present invention relates to a syringe.

Background Art

A prefilled syringe made of glass has been conventionally used as a medical packaging container for packaging and storing a chemical liquid in a sealed manner in clinical practice or the like. Such a prefilled syringe corresponds to an injector which is configured so that a chemical liquid necessary for therapy is packaged in a syringe in advance and a cap fitted to a cylinder tip (hereinafter, referred to as an "injection section".) of a syringe barrel through which the chemical liquid is injected is removed and then an injection needle is installed to the injection section in use. Such a prefilled syringe does not need any operation which involves once sucking a chemical liquid stored in an ampule or a vial by a syringe. Thus, use of such a prefilled syringe is expected to prevent incorporation of foreign substances into a chemical liquid, enhance efficiency of any medical work, prevent medical accidents such as incorrect administration of a chemical liquid, and the like. Conventionally, glass has been used in a syringe barrel or the like for use in such a prefilled syringe.

Such a prefilled syringe made of glass, however, has the problems of, for example, elution of a sodium ion or the like into a content liquid in a container during storage, generation of a fine substance called flakes, contamination of a metal for coloration in the case of use of a light-blocking glass container colored by such a metal, and easy cracking due to impact of dropping or the like. Such a prefilled syringe also has the problem of being increased in weight due to a large specific gravity of glass.

In order to solve the above problems, a technique using any plastic light in weight as compared with glass, as a replacement of glass for use in a syringe barrel, has been studied. There has been proposed, as a prefilled syringe using such plastic, a container for a prefilled syringe which has a layered structure of a resin layer and an oxygen barrier layer and which is excellent in oxygen barrier properties (see, for example, Patent Literature 1 below.).

Citation List

Patent Literature

Patent Literature 1: Japanese Patent Laid-Open No. 2016-019619

WO 2013/071138 A1 discloses features falling under the preamble of claim 1. US 2016/017490 A1, JP 2016/019619 A, JP 2006/045528 A, JP H10-314305 A, WO 2011/143509 A1, EP 2 957 310 A1, and EP 2 796 159 A1 are further prior art.

Summary of Invention

Technical Problem

Many techniques for an enhancement in oxygen barrier properties of a plastic prefilled syringe as described above have been proposed. On the other hand, there is a need for such a prefilled syringe which is not only excellent in sealing properties against a chemical liquid and gas in storage, but also excellent in slidability between the inner surface of a syringe barrel and a gasket in use.

The present invention has been made in order to solve the above problems, and an object thereof is to provide a syringe barrel which can exhibit excellent sealing properties against a liquid and gas and excellent slidability with respect to a gasket, and a syringe using the syringe barrel.

Solution to Problem

The present inventors have found that a prefilled syringe which can exhibit not only excellent sealing properties against a liquid and gas, but also excellent slidability with respect to a gasket by allowing the surface roughness of the inner surface of a syringe barrel to fall within a certain range, and thus have completed the present invention.

The invention is defined by claim 1.

Advantageous Effects of Invention

According to the present invention, a syringe barrel which can exhibit excellent sealing properties against a liquid and gas and excellent slidability with respect to a gasket, and a syringe using the syringe barrel can be provided.

Brief Description of Drawings

[Figure 1] Figure 1 is a schematic view illustrating a configuration of a syringe of the present embodiment in storage.
[Figure 2] Figure 2 is a schematic view for describing a layered structure of a cylindrical main body.
[Figure 3] Figure 3a is a plan view of a gasket 20 in Figure 3b, observed from upper side of the drawing, and Figure 3b is a cross-sectional view of the gasket 20 along with a sliding direction.
[Figure 4] Figure 4 is an image of an inner surface of a syringe barrel in Example 1, observed by an atomic force microscope.
[Figure 5] Figure 5 is an image of an inner surface of a syringe barrel in Comparative Example 1, observed by an atomic force microscope.

Description of Embodiments

Hereinafter, an embodiment (hereinafter, simply referred to as the "present embodiment".) for carrying out the present invention will be described in detail. The following present embodiment is illustrative of the present invention, and is not intended to limit the present invention to the following content. The present invention can be appropriately modified and carried out within the scope thereof.

<Configuration of syringe>

A configuration of a syringe using a syringe barrel of the present embodiment is described. It is noted that the following configuration corresponds to one aspect representing the syringe barrel of the present embodiment and a syringe including the syringe barrel and that the syringe barrel and the syringe of the present invention are not intended to be limited to the following configuration.

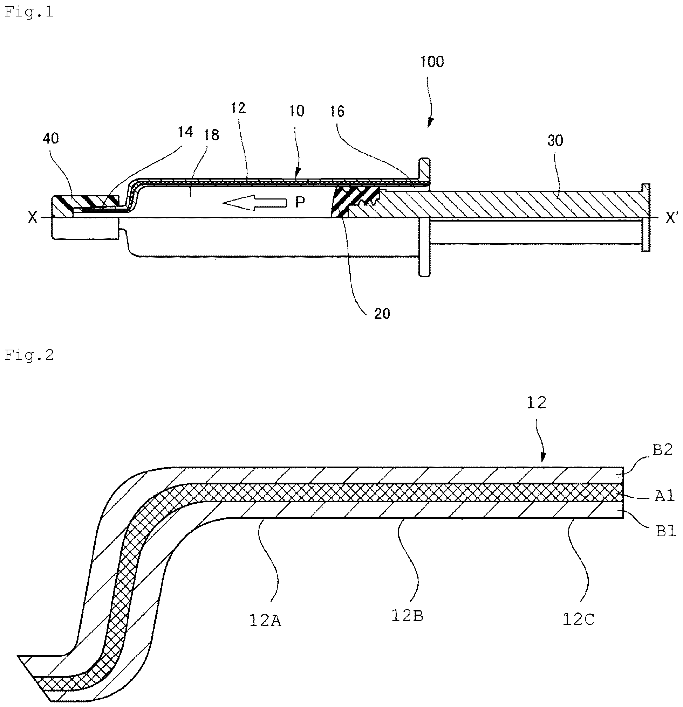

Figure 1 is a schematic view illustrating a configuration of a syringe of the present embodiment, in storage. As illustrated in Figure 1, a syringe 100 includes a syringe barrel 10, a gasket 20, a plunger 30, and a cap 40. The syringe 100 is a so-called prefilled syringe which accommodates a chemical liquid 18 in a sealed manner.

A syringe barrel 10 has a cylindrical main body 12, as illustrated in Figure 1, and accommodates the chemical liquid 18. The cylindrical main body 12 is a cylindrical body having the same diameter in the direction of a central axis indicated by a straight line XX' in Figure 1. The cylindrical main body 12 is provided with an injection section 14 for injection (discharging) of the chemical liquid 18 at a tip thereof and is provided with an insertion section 16 into which the gasket 20 is inserted, at other end.

As illustrated in Figure 2, the cylindrical main body 12 has a layered structure. Figure 2 is a schematic view for describing the layered structure of the cylindrical main body 12. The cylindrical main body 12 in the present embodiment has a three-layered structure where an oxygen barrier layer A1 is laminated between resin layers B1 and B2 (skin layer) so as to be an intermediate layer (core layer). While each material for use in the resin layers and the oxygen barrier layer is described below, the cylindrical main body 12 is preferably formed so that at least the resin layer B1 forming the inner surface (innermost layer) includes a cycloolefin-based polymer. The syringe 100 of the present embodiment is excellent in gas barrier properties because the syringe barrel 10 includes the oxygen barrier layer A1.

As illustrated in Figure 1, the cap 40 is fitted to the injection section 14 of the cylindrical main body 12 in storage of the syringe 100. The cap 40 is removed and an injection needle is installed to the injection section 14 in use of the syringe 100. The injection section 14 is formed from a section smaller in diameter than the cylindrical main body 12, and can also be formed so as to have a taper shape.

The gasket 20 is installed to one end of the plunger 30, and is disposed so as to be slidable in the syringe barrel 10 along with the straight line XX' in Figure 1. As described above, the gasket 20 is inserted through the insertion section 16 of the syringe barrel 10, and is pushed and moved in a direction (namely, a direction indicated by an arrow P in Figure 1) from the insertion section 16 toward the injection section 14 in use of the syringe 100. The gasket 20 is slid in the direction indicated by the arrow P, thereby allowing the chemical liquid 18 in the syringe barrel 10 to be pressurized in such a direction. As described above, the injection needle whose illustration is omitted is connected to the injection section 14 in use of the syringe 100, and the chemical liquid 18 pressurized by the gasket 20 is injected through the injection needle via the injection section 14.

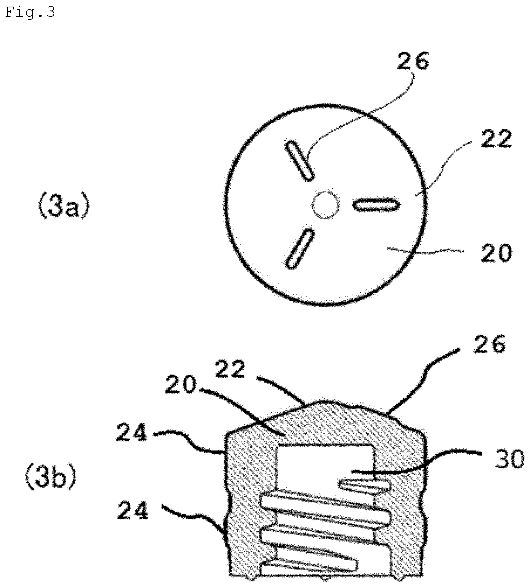

Next, the surface roughness of an inner surface of the cylindrical main body 12 of the syringe barrel 10 is described with reference to Figure 2. In the present embodiment, the surface roughness of each inner surface (sequentially referred to as inner surface 12A, inner surface 12B, and inner surface 12C toward the insertion section from a tip at which the injection section 14 is provided, in Figure 2.) of the cylindrical main body 12 is 4 nm to 25 nm in terms of the arithmetic average roughness (Ra). The surface roughness of each inner surface of the cylindrical main body 12 is less than 4 nm in terms of the arithmetic average roughness (Ra), thereby causing inferior slidability of the gasket 20 with respect to each inner surface of the cylindrical main body 12 (namely, a load required for pushing the plunger in sliding is increased). The surface roughness of each inner surface of the cylindrical main body 12 is more than 25 nm in terms of the arithmetic average roughness (Ra), thereby causing reduced sealing properties against a liquid and gas enclosed in the syringe barrel 10. The surface roughness of each inner surface of the cylindrical main body 12 (arithmetic average roughness (Ra)) is preferably 4 nm to 20 nm, further preferably 5 nm to 10 nm from the viewpoint of a further enhancement (reduction) in slidability between each inner surface of the cylindrical main body 12 and a sliding contact section 24 (see Figure 3), described below, of the gasket 20. The arithmetic average roughness (Ra) of each inner surface of the cylindrical main body 12 of the syringe barrel 10 can be determined by, for example, observing a surface profile with an apparatus such as a surface profile measuring instrument, a laser microscope, or an atomic force microscope, and thereafter performing calculation according to the expression described in JIS B 0601. Specifically, the arithmetic average roughness can be calculated according to a method shown in Examples below. The method of controlling the surface roughness of each inner surface of the cylindrical main body 12 is not particularly limited, and examples include a method involving setting the surface roughness of a mold for use in formation of the cylindrical main body 12, to a desired range and controlling the surface roughness of each inner surface of the cylindrical main body 12 within such a desired range. Examples of the method of controlling the surface roughness of the mold include a method involving adjusting the material of a polishing material, the size of an abrasive grain, the polishing load, and the polishing time in a step of polishing the mold.

The surface roughness of at least a portion of each inner surface of the cylindrical main body 12, the portion being in contact with the sliding contact section 24 of the gasket 20, may fall within the range from 4 nm to 25 nm in terms of the arithmetic average roughness (Ra), and the surface roughness of the entire inner surface may fall within the range. The surface roughness of each inner surface of the cylindrical main body 12 may be uniform in a circumferential direction or longitudinal direction (a direction indicated by an arrow P in Figure 1) or may be differential in such a direction with falling within the range from 4 nm to 25 nm in terms of the arithmetic average roughness (Ra). For example, the differences in surface roughness of the inner surface 12A located closer to the injection section, the inner surface 12B located at the center portion, and the inner surface 12C located closer to the insertion section, of the cylindrical main body 12, may be about ± 1 to 10 nm in terms of the arithmetic average roughness (Ra).

Hereinafter, each member will be described.

<Syringe barrel>

The cylindrical main body forming the syringe barrel of the present embodiment has a layered structure. The layered structure, but is not particularly limited, preferably has at least one oxygen barrier layer (hereinafter, sometimes simply referred to as "layer A") containing a thermoplastic resin (a) and at least one resin layer (hereinafter, sometimes simply referred to as "layer B") containing a thermoplastic resin (b). The layer configuration of the cylindrical main body is not particularly limited, and such layer A and layer B are not particularly limited in terms of the number and the type thereof. Such a layer A may be referred to as a "core layer" and such a layer B may be referred to as a "skin layer". For example, a three-layer structure can be adopted where at least two layers (layer B) containing a thermoplastic resin (b) are provided and the oxygen barrier layer (layer A) is disposed between such two layers (layer B) containing a thermoplastic resin (b) (B/A/B structure). That is, the cylindrical main body in the present embodiment may have an A/B configuration made of one layer A and one layer B or a three-layer configuration like a B/A/B structure made of one layer A and two layers B, as described above. The cylindrical main body may have a five-layer configuration, B1/B2/A/B2/B1, made of one layer A and four layers B of two layers B1 and two layers B2. The cylindrical main body in the present embodiment may include, if necessary, any layer such as an adhesion layer (layer AD), and may have, for example, a seven-layer configuration, B1/AD/B2/A/B2/AD/B1. The layer configuration of the cylindrical main body preferably has at least three layers as in a B/A/B structure. More specifically, the cylindrical main body preferably has a layered structure of three or more layers, in which an intermediate layer including a composition including a polyester and a transition metal catalyst, or a polyamide is provided as the oxygen barrier layer.

<Oxygen barrier layer (layer A)>

The oxygen barrier layer (layer A) may be obtained by using, as the thermoplastic resin (a), a strict oxygen barrier resin (so-called passive barrier resin) merely having the property of allowing oxygen to hardly penetrate, or an oxygen absorbing resin composition (so-called active barrier resin composition) which has oxygen absorbing and which can absorb oxygen penetrating from the external to thereby prevent oxygen from penetrating into the internal. Examples of the oxygen barrier resin include polyamide, and examples of the oxygen absorbing resin composition include a composition including a polyester and a transition metal catalyst.

The glass transition temperature ($Tg^1$) of the thermoplastic resin (a) is preferably 50°C to 160°C, more preferably 55°C to 150°C, further preferably 60°C to 140°C. The glass transition temperature ($Tg^1$) of the thermoplastic resin (a) falls within the temperature range, thereby enabling a syringe barrel favorable in heat resistance and formability to be produced.

(Composition including polyester and transition metal catalyst)

The polyester is not particularly limited, and examples include a composition having a constitutional unit containing a tetralin ring.

-Tetralin ring-containing polyester compound-

In the case where the oxygen barrier layer (layer A) includes a composition including a polyester and a transition metal catalyst, the layer can be formed by using, for example, a composition including a polyester compound having a tetralin ring as a constitutional unit. Any composition described in International Publication No. WO 2013/077436 can also be used as such a composition including a polyester compound (a1) having a tetralin ring as a constitutional unit, and a transition metal catalyst.

The polyester compound and the transition metal catalyst can be mixed by a known method, and are preferably kneaded by an extruder. Thus, an oxygen barrier resin composition favorable in dispersibility can be obtained. As long as the effects of the present embodiment are not impaired, any other additive(s), for example, a desiccant, a pigment, a dye, an antioxidant, a slip agent, an antistatic agent, and a stabilizer; a filler such as calcium carbonate, clay, mica, and silica; and a refresher may be added to such an oxygen barrier resin composition, and various materials can be mixed therewith, without any limitation to such additives listed above.

(Polyamide)

As described above, the oxygen barrier layer (layer A) may be a layer including a polyamide. The polyamide is not particularly limited, and, for example, a polyamide resin (a2) can be used which includes a constitutional unit derived from diamine and a constitutional unit derived from dicarboxylic acid, in which the constitutional unit derived from diamine includes 70 mol% or more of a constitutional unit derived from m-xylylenediamine and the constitutional unit derived from dicarboxylic acid includes 30 to 60 mol% of a unit structure derived from at least one of an $\alpha$-linear aliphatic dicarboxylic acid and an $\omega$-linear aliphatic dicarboxylic acid each having 4 to 20 carbon atoms, and 70 to 40 mol% of a constitutional unit derived from isophthalic acid.

The polyamide resin (a2) is an amorphous resin. The amorphous resin is used to thereby enable transparency to be enhanced. Furthermore, the polyamide resin (a2) is also excellent in barrier properties after a thermal sterilization treatment. The polyamide resin (a2) preferably includes a calcium atom. The resin includes a calcium atom to thereby enable transparency after a heating treatment to be more enhanced. The polyamide resin (a2) more preferably includes a phosphorus atom at a proportion of 20 to 200 ppm by mass and a calcium atom at a molar ratio of phosphorus atom: calcium atom of 1:0.3 to 0.7. Such a configuration is adopted to thereby provide a syringe barrel higher in transparency after a heating treatment and lower in YI value (degree of yellowness) after a heating treatment. Such a calcium atom is preferably derived from calcium hypophosphite.

The polyamide resin (a2) which can be here used is any polyamide resin described in International Publication No. WO 2017/090556. The oxygen permeation coefficient of the polyamide resin (a2) is preferably a value of 0.5 mL mm/($m^2$ day atm) or less, as obtained by the procedure according to JIS K 7126.

The oxygen barrier layer (layer A) may include any polyamide resin other than the polyamide resin (a2), and such any polyamide resin other than the polyamide resin (a2) may be an amorphous resin or a crystalline resin and is preferably an amorphous resin.

(Other)

The oxygen barrier layer (layer A) may, if necessary, further contain a radical generator and a photoinitiator in order to enhance oxygen barrier performance. The oxygen barrier resin composition can also be kneaded together with any thermoplastic resin other than the above thermoplastic resin by an extruder as long as the objects of the present embodiment are not impaired. Such radical generator, photoinitiator, and other thermoplastic resin which can be used are each any known product. Examples of the radical generator include N-hydroxyimide compounds such as N-hydroxysuccinimide and N-hydroxymaleimide. Examples of the photoinitiator include benzophenone and a derivative thereof, a thiazine dye, a metal porphyrin derivative, and an anthraquinone derivative. Examples of such other thermoplastic resin include polyolefin typified by polyethylene, an ethylene-vinyl compound copolymer, a styrene-based resin, a polyvinyl compound, polyamide, polyester, and polycarbonate.

The content of the composition including a polyester and a transition metal catalyst or the polyamide resin in the oxygen barrier layer (layer A) is not particularly limited, and is preferably 50% by mass or more, more preferably 70% by mass or more, particularly preferably 90% by mass or more. Such a range can allow oxygen barrier performance to be more enhanced than the range of less than 50% by mass. Such a compound may be each used singly or in combination.

The thickness of the oxygen barrier layer (layer A) is not particularly limited, and is preferably 1 to 1000 $\mu$m, more preferably 2 to 800 $\mu$m, further preferably 5 to 700 $\mu$m. The thickness of the layer A is controlled within the range, thereby not only enabling oxygen barrier properties of the layer A to be more enhanced, but also enabling economic efficiency to be prevented from being impaired. The oxygen permeation coefficient of the oxygen barrier layer (layer A) is preferably a value of 0.5 mL/(mm m$^2$ day atm) or less, as obtained by the procedure according to JIS K 7126.

<Layer (layer B) containing thermoplastic resin (b)>

The layer B in the present embodiment is a layer containing a thermoplastic resin (b). Unless particularly noted, a layer B1 and a layer B2 are collectively referred to as "layer B". Similarly, in the case where thermoplastic resins (b1) and (b2) are represented, such resins are also collectively referred to as "thermoplastic resin (b)". The thermoplastic resin (b) is a thermoplastic resin other than the thermoplastic resin (a). The content of the thermoplastic resin (b) in the layer B is not particularly limited, and is preferably 70 to 100% by mass, more preferably 80 to 100% by mass, further preferably 90 to 100% by mass. In the case of an aspect where the layer containing the thermoplastic resin (b) is formed from a plurality of layers like layers B1 and B2, the content of the thermoplastic resin (b) in the layer B, here mentioned, refers to the content of the thermoplastic resin (b) in each layer.

Such an oxygen barrier multi-layer body in the present embodiment may include a plurality of the layers B, as described above. In the case where such an article includes a plurality of such layers B, the respective configurations of such layers B may be the same as or different from each other. The thickness of the layer B can be appropriately determined depending on the application. In general, in the case where the oxygen barrier multi-layer body is used in the syringe barrel, the thickness of such one layer B is preferably 5 $\mu$m to 1000 $\mu$m, more preferably 10 $\mu$m to 800 $\mu$m, further preferably 20 $\mu$m to 500 $\mu$m from the viewpoint that various physical properties required for the syringe barrel, for example, strength such as dropping resistance, and flexibility are ensured.

The thermoplastic resin (b) is not particularly limited and any thermoplastic resin other than the thermoplastic resin (a) can be used. Specific examples of the thermoplastic resin (b) include known polyolefin, polyester, polyamide, ethylene-vinyl alcohol copolymer, plant-derived resin and fluorine-based resin. The thermoplastic resin (b) preferably includes at least one selected from the group consisting of such resins. In particular, polyolefin is preferable. More specific and suitable examples include a copolymer obtained from norbornene and an olefin such as ethylene, as raw materials; and a cycloolefin copolymer (COC) which is a copolymer obtained from tetracyclododecene and an olefin such as ethylene, as raw materials. A cycloolefin polymer (COP) which is a polymerized product obtained by subjecting norbornene to ring-opening and hydrogenation is also particularly preferable. Such COC and COP which can be used are, for example, any polymers described in Japanese Patent Laid-Open No. 5-300939 or Japanese Patent Laid-Open No. 5-317411.

A commercially available product can be used as COC. For example, Apel (registered trademark) manufactured by Mitsui Chemicals, Inc. is commercially available. A commercially available product can be used as COP. For example, ZEONEX (registered trademark) manufactured by Zeon Corporation is commercially available. COC and COP are particularly preferable materials because such materials exhibit characteristics of polyolefin resins, for example, chemical properties such as heat resistance and light resistance, and chemical resistance, and exhibit characteristics of amorphous resins, for example, physical properties such as mechanical properties, melting, flow properties, and dimension accuracy.

The glass transition temperature ($Tg^2$) of the thermoplastic resin (b) is preferably -10 to +20°C, more preferably -5 to +15°C, further preferably $\pm$0 to +10°C relative to the glass transition temperature ($Tg^1$) of the thermoplastic resin (a). The glass transition temperature ($Tg^2$) of the thermoplastic resin (b) falls within the temperature range relative to the glass transition temperature ($Tg^1$) of the thermoplastic resin (a), thereby enabling a molded product favorable in appearance to be produced in formation of a multi-layer body with the thermoplastic resin (a).

<Other>

The cylindrical main body of the syringe barrel may further include any layer depending on the desired performance, in addition to the oxygen barrier layer (layer A) and the layer (layer B) containing the thermoplastic resin (b). Examples of such any layer include an adhesion layer (layer AD). For example, in the case of a configuration where the layer B is formed on the layer A, a configuration (layer A/layer AD/layer B) may be adopted where the layer B is formed on the layer A with the layer AD being interposed.

In the case where there is not obtained any practical interlayer adhesion strength between two adjacent layers in the cylindrical main body of the syringe barrel, the adhesion layer (layer AD) can be provided between the two layers. The adhesion layer preferably includes a thermoplastic resin having adhesiveness. Examples of the thermoplastic resin having adhesiveness include an acid-modified polyolefin resin obtained by modifying a polyolefin-based resin such as polyethylene or polypropylene by an unsaturated carboxylic acid such as acrylic acid, methacrylic acid, maleic acid, maleic anhydride, fumaric acid or itaconic acid; and a polyester-based thermoplastic elastomer including a polyester-based block copolymer as a main component. The adhesion layer which is preferably used is a modified product of the same resin as the thermoplastic resin used in the layer B from the viewpoint of adhesiveness. The thickness of the adhesion layer is preferably 2 to 100 $\mu$m, more preferably 5 to 90 $\mu$m, further preferably 10 to 80 $\mu$m from the viewpoint that not only practical adhesion strength is exhibited, but also processability is ensured.

The method of producing the syringe barrel of the present embodiment is not particularly limited, and the syringe barrel can be produced according to a usual injection molding method. For example, such production can be made by injecting a material for formation of the layer A and a material for formation of the layer B into a cavity through a mold hot runner of each injection cylinder by use of a molding machine provided with two or more injection machines, and a mold for injection, to thereby produce a syringe barrel whose shape corresponds to the shape of the mold for injection.

A syringe barrel of a three-layer structure B/A/B can be produced by first injecting a material for formation of the layer B from an injection cylinder, then injecting a material for formation of the layer A from another injection cylinder, at the same time as injection of a resin for formation of the layer B, and then injecting an required amount of the resin for formation of the layer B to fill a cavity.

A syringe barrel of a five-layer structure B/A/B/A/B can be produced by first injecting a material for formation of the layer B, then singly injecting a material for formation of the layer A, and finally injecting an required amount of the material for formation of the layer B to fill a mold cavity.

A syringe barrel of a five-layer structure B1/B2/A/B2/B1 can be produced by first injecting a material for formation of the layer B1 from an injection cylinder, then injecting a material for formation of the layer B2 from another injection cylinder, at the same time as injection of a resin for formation of the layer B1, then injecting a resin for formation of the layer A at the same time as injection of respective resins for formation of the layer B1 and the layer B2, and injecting an required amount of the resin for formation of the layer B1 to fill a cavity.

A multi-layer molded article may also be obtained according to a compression molding method. For example, such a molded article is obtained by providing an oxygen barrier resin agent in a thermoplastic resin melt, feeding the molten mass to a male mold and also compressing it by a female mold, and cooling and solidifying such a compression-molded product.

A neck portion of the resulting molded article may be subjected to a heat treatment and thus crystallized at this stage in order to impart heat resistance to the neck portion. The crystallinity is preferably 30 to 50%, more preferably 35 to 45%.

The molded article may be formed so as to have a desired container shape according to any molding procedure such as extrusion or compression molding (sheet molding or blow molding).

<Gasket>

The syringe barrel of the present embodiment is combined with a suitable gasket to thereby exhibit favorable air-tightness, liquid-tightness, and slidability. The syringe barrel of the present embodiment can be combined with a conventionally known gasket appropriately selected.

A conventionally known gasket suitably used with the syringe barrel of the present embodiment can be a rubber gasket called "laminate gasket" including an inactive film layer on the surface of at least one of the above wetted section and sliding contact section from the viewpoint of an eluted product into the chemical liquid, slidability, a reduction in the amount of a silicone compound for coating, and the like. An inactive film excellent in slidability can be used as the above inactive film, and can be laminated on the surface of the gasket. The laminate gasket can be used to thereby inhibit a rubber component from being transferred into the chemical liquid and reduce the amount of the silicone compound with slidability being retained.

The rubber serving as a raw material of a rubber composite forming the main body of the gasket, which is here used, is, for example, butyl-based rubber, isoprene rubber, butadiene rubber, styrene/butadiene rubber, natural rubber, chloroprene rubber, nitrile-based rubber such as acrylonitrile/butadiene rubber, hydrogenated nitrile-based rubber, norbornene rubber, ethylene/propylene rubber, ethylene-propylene-diene rubber, acrylic rubber, ethylene-acrylate rubber, fluororubber, chlorosulfonated polyethylene rubber, epichlorohydrin rubber, silicone rubber, urethane rubber, polysulfide rubber, phosphazene rubber, or 1, 2-polybutadiene.

These may be used singly or in combinations of two or more kinds thereof.

The rubber for use in the rubber composite is not limited to the above materials, and butyl-based rubber is preferable because of excellent gas permeation resistance and water-vapor permeation resistance. A known compound may be used as the butyl-based rubber, and examples include isobutylene-isoprene copolymerized rubber, halogenated isobutylene-isoprene copolymerized rubber (hereinafter, referred to as "halogenated butyl rubber"), or a modified product thereof. Examples of the modified product include a brominated product of a copolymer of isobutylene and p-methylstyrene. In particular, halogenated butyl rubber is more preferable and chlorinated butyl rubber or brominated butyl rubber is further preferable in terms of easiness of crosslinking.

The inactive film layer preferably includes a fluororesin. The inactive film for surface formation of the laminate gasket is not particularly limited, and is preferably at least one fluororesin selected from the group consisting of polytetrafluoroethylene (PTFE), modified polytetrafluoroethylene (modified PTFE; a copolymer of a 4F monomer and a trace amount of perfluoroalkoxide), a tetrafluoroethylene/ethylene copolymer (ETFE)), a tetrafluoroethylene/perfluoroalkyl vinyl ether copolymer (PFA) and polychlorotetrafluoroethylene (PCTFE), and/or an olefin-based resin, particularly preferably polytetrafluoroethylene (PTFE), from the viewpoint that favorable chemical resistance is obtained.

The shape of the laminate gasket can be a conventionally known shape. The structure of the gasket 20 is described with reference to Figure 3. Figure 3 illustrates the structure of the gasket 20 in the present embodiment, Figure 3a is a plan view of a gasket 20 in Figure 3b, observed from upper side of the drawing, and Figure 3b is a cross-sectional view of the gasket 20 along with a sliding direction. The gasket 20, while illustrated in Figure 3b with being partially omitted, is installed to a tip of the plunger 30, as illustrated in Figure 1, and is configured so that the gasket 20 can be slid with being in contact with the inner surface of the syringe barrel 10 by pushing the plunger 30 in an arrow P direction in Figure 1 by a user of the syringe 100. The gasket 20 has a wetted section 22 in its front section (end surface of the gasket 20 in an upper direction of the drawing in Figure 3b), and is in contact with the chemical liquid 18 in the syringe barrel 10. As illustrated in Figure 3, the gasket 20 has a sliding contact section 24 having a circular rib structure. The sliding contact section 24 of the gasket 20 is in close contact with the inner surface of the syringe barrel 10, thereby allowing the chemical liquid 18 to be stored in the syringe barrel 10 in a sealed manner. An inactive film layer including polytetrafluoroethylene or the like is provided on the surface of at least one of the wetted section 22 and the sliding contact section 24 of the gasket 20, although illustration thereof is omitted. A protrusion section 26 can also be provided on the wetted section 22 of the gasket 20.

The gasket 20 may have only one circular rib or a plurality of circular ribs each serving as the sliding contact section 24. For example, the gasket 20 illustrated in Figure 3 has two circular ribs present therein, including a front circular rib and a rear circular rib. The number of such circular ribs may be one, or three or more.

The shape of the sliding contact section 24 is not particularly limited. In the case where a plurality of circular ribs are each present as the sliding contact section 24, the length of the sliding contact section in the entire gasket relative to the length of the sliding contact section of the front circular rib is not particularly limited, and the radius of curvature of the front corner section of the front circular rib and the radius of curvature of the rear corner section are not particularly limited. The cross section of the sliding contact section of the front circular rib may be increased, decreased or unchanged in diameter on a substantially straight line along with the longitudinal direction of the gasket. The diameter of the front circular rib may be larger or smaller than the diameter of the circular rib other than the front circular rib, or such diameters may be substantially the same.

The rate of compression of the circular rib relative to the inner diameter of the syringe is preferably 2 to 8%, more preferably 3 to 7%. A rate of compression of less than 2% tends to result in inferior liquid-tightness and air-tightness, and a rate of compression of more than 8% tends to make tapping onto the barrel difficult, causing a wrinkle to occur on the film on the circular rib surface to also result in inferior liquid-tightness and high sliding resistance. The rate of compression is calculated by the following expression. The rate of compression may be uniform or different along with the longitudinal direction (direction indicated by an arrow P in Figure 1) of the cylindrical main body 12. For example, the rate of compression may also be designed so as to be gradually smaller toward the injection section from the insertion section.

```
Rate of compression (%) = ((Diameter of circular

rib) - (Inner diameter of barrel))/(Diameter of circular

rib) × 100
```

The laminate gasket can also be obtained by forming the shape of the gasket and thereafter polishing the surface of the laminate film in a circumferential direction. The surface of the film after such formation can be polished in a circumferential direction, thereby allowing fine scuff in a sliding direction, generated in forming and releasing, to disappear, and contributing to enhancements in air-tightness and liquid-tightness. Examples of the polishing method include a method involving rubbing with a polymer cloth and a method involving polishing with a base material using a fine abrasive particle. Examples of the polymer cloth include a cotton cloth and a rayon cloth. Examples of the fine abrasive particle include alumina, silicon carbide, and diamond. The direction of such polishing performed is preferably a circumferential direction. In the case where the gasket has a plurality of circular rib structures, such polishing is preferably performed at least throughout the front circular rib. The degree of such polishing can be expressed with the ratio of the arithmetic average roughness Ra// in the circumferential direction and the arithmetic average roughness Ra⊥ in the sliding direction, of the surface of the gasket, or the difference between the maximum height Ry// in the circumferential direction and the maximum height Ry⊥ in the sliding direction, of the surface of the gasket.

The arithmetic average roughness (Ra) of the surface of the sliding contact section of the gasket is preferably 20 nm to 100 nm, further preferably 25 nm to 70 nm, particularly preferably 25 nm to 60 nm from the viewpoint of slidability with respect to the inner surface of the syringe barrel and sealing properties.

The laminate gasket can also be coated with a silicone compound, mainly on the sliding contact section thereof. The silicone compound is not particularly limited as long as the compound is a compound having a siloxane bond. The silicone compound preferably has a structure including a T unit and a Q unit in a silicone backbone, and preferably encompasses a silicone compound having branched structure-crosslinked structure. Examples of such a silicone compound having a structure including a T unit and a Q unit in a silicone backbone include various silicone resins such as a methylsilicone resin, a phenylsilicone resin, an epoxy-modified silicone resin, an acrylic-modified silicone resin and a polyester-modified silicone resin, and polysilsesquioxane such as polyphenylsilsesquioxane, polymethylsilsesquioxane and polymethylphenylsilsesquioxane.

A preferable example of the silicone compound is a mixture of a silicone compound having branched structure-crosslinked structure having a T unit and a Q unit in a silicone backbone and a linear silicone compound having a D unit as a main structure in a silicone backbone. More specifically, a mixture of trimethylsiloxysilicic acid and polydimethylsiloxane, and a mixture of polymethylsilsesquioxane and polydimethylsiloxane are desirable.

The mixing ratio in the mixture of the silicone compound having branched structure-crosslinked structure having a T unit and a Q unit in a silicone backbone and the linear silicone compound having a D unit as a main structure in a silicone backbone is not particularly limited, and the compounding ratio of the silicone compound having branched structure-crosslinked structure having a T unit and a Q unit in a silicone backbone is preferably 5 to 50% by weight, more preferably 10 to 40% by weight, further preferably 20 to 30% by weight. The compounding ratio of the silicone compound having branched structure-crosslinked structure having a T unit and a Q unit in a silicone backbone is so high that sliding resistance to the inner surface of the barrel of the gasket can be higher, and the compounding ratio is so low that the surface of the gasket is not uniformly coated in some cases.

The above description provides an example of a conventionally known gasket suitably used in the syringe barrel of the present embodiment, and the gasket suitably used in the syringe barrel of the present embodiment is not limited to the above example.

<Cap>

The cap in the present embodiment is installed to the injection section of the syringe barrel. A material having an oxygen permeation coefficient of 300 mL mm/($m^2$ day atm) or less is preferably used as the material for formation of the cap. Furthermore, the oxygen permeation coefficient of the material for formation of the cap is preferably 200 mL mm/($m^2$ day atm) or less, more preferably 150 mL mm/($m^2$ day atm) or less, particularly preferably 100 mL mm/($m^2$ day atm) or less. Examples include butyl rubber made of a copolymer of isobutylene and a small amount of isoprene, and bromobutyl rubber and chlorobutyl rubber each obtained by halogenation of butyl rubber, and bromobutyl rubber and chlorobutyl rubber are preferable. The thickness of the cap of a portion of the syringe barrel, the portion being in contact with the injection section, is preferably 300 to 20000 μm, more preferably 500 to 10000 μm, particularly preferably 1000 to 5000 μm from the viewpoint of oxygen barrier performance. The thickness of the cap of a portion of the syringe barrel, the portion covering the opening of the injection section, is preferably 500 to 30000 μm, more preferably 1000 to 20000 μm, particularly preferably 2000 to 10000 μm from the viewpoint of oxygen barrier performance.

<Plunger>

The plunger in the present embodiment is installed to the gasket, and the plunger is pushed to thereby transfer the gasket. The plunger which can be used is a plunger for use in a common syringe, and the structure and the material of such a plunger are not limited.

<Prefilled syringe>

Examples of one aspect of the prefilled syringe include an injector (syringe) configured so that a chemical liquid or the like is accommodated in a syringe equipped with the above syringe barrel, gasket, cap and plunger and a tip of the barrel is opened and an injection needle is installed thereto in use.

The chemical liquid is not particularly limited, and examples include vitamin supplements such as vitamin A, vitamin B2, vitamin B12, vitamin C, vitamin D, vitamin E and vitamin K, alkaloid such as atropine, hormones such as adrenaline and insulin, sugars such as glucose and maltose, antibiotics such as ceftriaxone, cephalosporin and cyclosporine, and benzodiazepine-based agents such as oxazolam, flunitrazepam, clotiazepam and clobazam, in terms of the effects of the present embodiment. The syringe of the present embodiment, in the case of accommodating a chemical liquid or the like including such a compound, enables denaturation due to oxidation to be suppressed.

[Sterilization treatment]

A sterilization treatment of a container and/or such a chemical liquid can be performed in a manner suitable for the chemical liquid, before and after the chemical liquid is accommodated. Examples of the sterilization method include heat sterilization such as a hot-water treatment at 100°C or less, a pressurized hot-water treatment at 100°C or more, and an ultra-high temperature heating treatment at 130°C or more; sterilization with electromagnetic ray such as ultraviolet ray, microwave or gamma ray; a treatment with gas such as ethylene oxide; and sterilization with a chemical agent such as hydrogen peroxide or hypochlorous acid.

Examples

Hereinafter, the present invention will be described in more detail with reference to Examples and Comparative Examples, but the present invention is not limited thereto. Herein, NMR measurement was performed at room temperature, unless particularly noted.

[Examples 1 and 2, and Comparative Examples 1 and 2]

<Production of syringe>

(Synthesis Example of monomer)

An autoclave having an inner volume of 18 L was charged with 2.20 kg of dimethyl naphthalene-2,6-dicarboxylate, 11.0 kg of 2-propanol, and 350 g of a catalyst (product containing 50% by mass of water) including 5% of palladium carried on activated carbon. Next, the air in the autoclave was replaced with nitrogen and furthermore the nitrogen was replaced with hydrogen, and thereafter hydrogen was fed until the pressure in the autoclave reached 0.8 mPa. Next, a stirrer was driven to adjust the rotational speed to 500 rpm, the inner temperature was raised to 100°C over 30 minutes, and thereafter hydrogen was further fed to adjust the pressure to 1 mPa. Thereafter, such feeding of hydrogen was continued so that the pressure was kept at 1 mPa, depending on the pressure drop due to progression of the reaction. The pressure drop was terminated after 7 hours, and thus the autoclave was cooled to discharge unreacted remaining hydrogen and thereafter a reaction liquid was taken out from the autoclave. The reaction liquid was filtered to remove the catalyst, and thereafter 2-propanol was evaporated from the filtrate separated, by an evaporator. To the resulting crude product was added 4.40 kg of 2-propanol, and purification was made by recrystallization, thereby providing dimethyl tetralin-2,6-dicarboxylate at a yield of 80%. The melting point of dimethyl tetralin-2,6-dicarboxylate purified was 77°C. The analytical results of NMR are as follows.
1H-NMR (400 mhz cdcl3) δ 7.76-7.96 (2H m), 7.15 (1H d), 3.89 (3H s), 3.70 (3H s), 2.70-3.09 (5H m), 2.19-2.26 (1H m), 1.80-1.95 (1H m)

(Synthesis Example of polymer)

An apparatus for producing a polyester resin, equipped with a packed column type rectification column, a partial condenser, a total condenser, a cold trap, a stirrer, a heater and a nitrogen introduction tube, was charged with 504 g of dimethyl tetralin-2,6-dicarboxylate obtained in the Synthesis Example of monomer, 227 g of ethylene glycol, 0.027 g of tetrabutyl titanate, and 0.043 g of zinc acetate, and the mixture in the apparatus was heated to 230°C in a nitrogen atmosphere and subjected to a transesterification reaction. After the rate of reaction conversion of such a dicarboxylate component reached 90% or more, temperature rise and pressure drop were gradually performed over 90 minutes, and polycondensation was performed at 275°C and at 133 Pa or less for 1 hour, thereby providing tetralin ring-containing polyester compound (1) (hereinafter, also designated as "polyester compound".). The resulting polyester compound had a weight-average molecular weight of $2.8 \times 10^4$ and a number average molecular weight of $2.8 \times 10^4$, in terms of polystyrene, and had a glass transition temperature of 69°C, and no melting point was observed because the polyester compound was amorphous. The intrinsic viscosity was 0.92 dl/g.

(Production Example of oxygen absorbing resin composition)

A mixture obtained by dry blending cobalt (II) stearate with 100 parts by mass of the above-obtained polyester compound so that the amount of cobalt was 0.0002 parts by mass was fed to a twin screw extruder having two screws each having a diameter of 37 mm, at a rate of 12 kg/h, and molten and kneaded in a condition of a cylinder temperature of 210°C, and a strand was extruded through an extruder head and cooled, and thereafter pelletized, thereby providing an oxygen absorbing resin composition.

(Production Example of syringe barrel)

A material for formation of a skin layer (layer B) was injected from an injection cylinder in the following conditions and thereafter a material for formation of a core layer (layer A) was injected from another injection cylinder at the same time as injection of a resin for formation of a skin layer (layer B), and then a required amount of such a resin for formation of a skin layer (layer B) was injected to fill a cavity in an injection mold, thereby producing a syringe barrel having a three-layer configuration of B/A/B. The degree of grinding of the mold in contact with the inner surface of the syringe barrel was modulated to thereby modulate the surface roughness of the inner surface of the syringe barrel. The resin for formation of a skin layer (layer B), which was here used, was a cycloolefin polymer (trade name: ZEONEX 5000, manufactured by Zeon Corporation, glass transition temperature: 68°C). The resin for formation of a core layer (layer A), which was here used, was the above oxygen absorbing resin composition. A gate formed was cut by an ultrasonic fusion machine at a position where the diameter at a position of 0.75 mm from an end after cutting was a value prescribed according to ISO80369-7 ($\varphi$4.021 $\pm$ 0.051 mm).

(Shape of syringe barrel)

The amount of the content, according to ISO11040-6, was 1 ml (Long) or 2.25 ml. The syringe was produced by using an injection molding machine (Model: GL-150 manufactured by Sodick Co., Ltd., or Model: ASB-12N/10 manufactured by Nissei Asb Machine Co., Ltd.).

-Forming conditions of syringe barrel-

Temperature of injection cylinder for skin layer (layer B): 315°C
Temperature of injection cylinder for core layer (layer A): 280°C
Temperature of resin passage in injection mold: 300°C
Mold temperature: 30°C

(Gasket)

A unvulcanized rubber sheet including chlorinated butyl rubber, and PTFE were pasted, and molded with vulcanization adhesion by vacuum press at 175°C for 10 minutes, thereby providing a molded sheet where the shape of a gasket was formed. An unnecessary portion was cut off from the resulting molded sheet, thereby providing a gasket. The resulting gasket was washed, sterilized and dried, thereby providing a predetermined sterilized gasket. The resulting sterilized gasket was coated with a silicone compound according to a known method, thereby providing a predetermined gasket coated with a silicone compound.

Hereinafter, the gasket coated with a silicone compound is designated as a gasket, unless particularly noted.

[Performance evaluation of syringe]

The performance test of the resulting syringe was performed by measurement and evaluation according to the following methods and criteria.

[Slidability test]

The gasket was attached to the syringe barrel so that the amount of the content corresponded to the nominal volume of the syringe, and filled with pure water in an amount equal to the nominal volume of the syringe and left to still stand in a constant temperature room at 23°C for 12 hours. Thereafter, a needle and a plunger were attached, a tip of the barrel, and the gasket were secured to a constant temperature bending tensile tester (INSTRON (registered trademark) 5267), and a load necessary for pushing the plunger at a rate of 200 mm/min was measured. The needle here used was a needle according to a standard of 25 G × 1 inch.
The average load at a sliding distance of 10 mm to 25 mm was calculated as the average sliding resistance value. The results are shown in Table 1.

[Liquid-tightness test and air-tightness test]

The tests were performed according to JIS T3210:2011. The results are shown in Table 1.

<Liquid-tightness test (pressure test)>

Distilled water was sucked into the syringe barrel from which moisture was sufficiently wiped off, to each location corresponding to the nominal volume scale, three-quarters of the nominal volume scale and one-half of the nominal volume scale, the resultant was horizontally secured and a tip of the nozzle of the syringe barrel was sealed, and thereafter a plunger rod was clamped to the gasket. The presence of any water droplet dropping through a space between the gasket and the syringe barrel was confirmed in application of a pressure of 343 kPa to the plunger rod for 10 seconds. The following Table shows the number of syringe barrel(s) from which any water droplet dropped, among three syringe barrels.

<Air-tightness test (sucking test)>

The presence of the occurrence of any continuous air bubble through a space between the gasket and the syringe barrel was confirmed, in the case of sucking water to a location of one-quarter of the nominal volume scale of the syringe barrel, sealing a tip of the nozzle of the syringe barrel and thereafter pulling the plunger rod to the location of the nominal volume scale. The Table representing the results shows the number of syringe barrel(s) in which any continuous air bubble occurred, among three syringe barrels.

[Surface roughness]

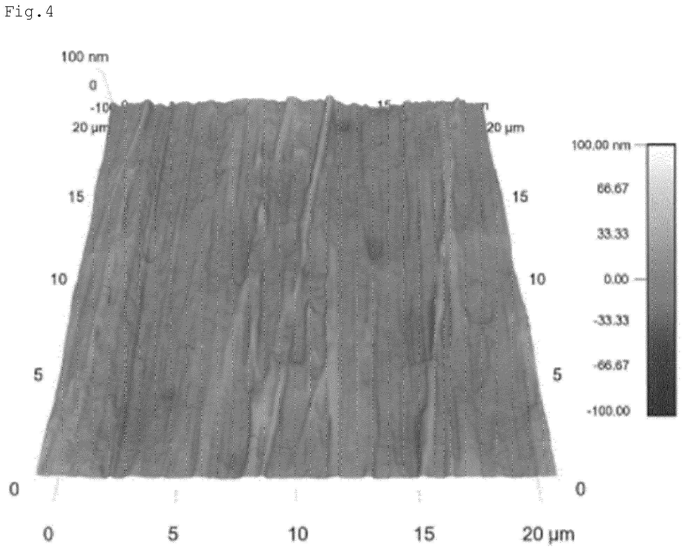

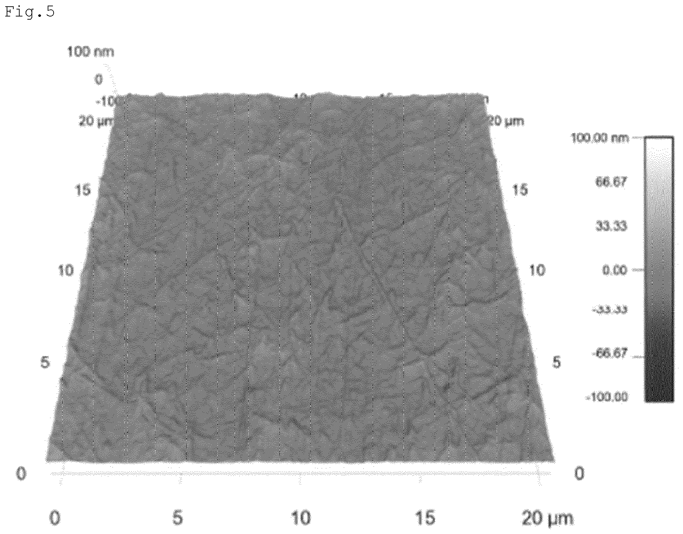

A piece of the syringe barrel cut was taken, and the surface profile of the inner surface of the barrel was measured. Such measurement was conducted using an atomic force microscope (MFP-3D-SA-J manufactured by Oxford Instruments). Respective images observed with the atomic force microscope, in Example 1 and Comparative Example 1, are shown in Figures 4 and 5. The arithmetic average roughness Ra was calculated from each of the resulting surface profiles. The surface profile was measured at respective three points of a flange section (see inner surface 12C in Figure 2), a center section (see inner surface 12B in Figure 2), and a front section (see inner surface 12A in Figure 2) of the syringe barrel, in an area of 20 μm × 20 μm with respect to each of such sections. The results are shown in Table 1.
The average arithmetic roughness (Ra) of the surface of the sliding contact section of the gasket was measured according to the same method. The results are shown in Table 1.

[Table 1]

| Items | Unit | Example 1 | Example 2 | Comparative Example 1 | Comparative Example 2 |
|---|---|---|---|---|---|
| Shape | - | 1 mL Long | 2.25 mL | 1 mL Long | 2.25 mL |
| Slidability | (N) | 5.6 | 7.0 | 9.2 | 10.5 |
| Liquid-tightness test | - | 0/3 | 0/3 | 0/3 | 0/3 |
| Air-tightness test | - | 0/3 | 0/3 | 0/3 | 0/3 |
| Arithmetic average roughness Ra of inner surface of syringe barrel | (nm) | 7.5 | 9.1 | 3.3 | 3.0 |

(continued)

| Items | Unit | Example 1 | Example 2 | Comparative Example 1 | Comparative Example 2 |
|---|---|---|---|---|---|
| Arithmetic average roughness Ra of surface of gasket | (nm) | 26 | 66 | 26 | 66 |

It was found from Table 1 that any syringe barrel having a surface roughness of the inner surface, of below 4 nm, in terms of the arithmetic average roughness (Ra) was inferior in slidability.

[Example 3]

A syringe barrel was produced in the same manner except that the following oxygen barrier resin composition was used instead of the oxygen absorbing resin composition in Example 1, and the syringe barrel was evaluated in the same manner except that a low surface tension liquid-tightness test described below was performed instead of the liquid-tightness test-air-tightness test in Example 1. The evaluation results are shown in the following Table.

(Production Example of oxygen barrier resin composition)

A reaction container equipped with a stirrer, a partial condenser, a total condenser, a thermometer, a dropping funnel and a nitrogen introduction tube was loaded with 6,000 g (41.06 mol) of adipic acid, 6,821 g (41.06 mol) of isophthalic acid, 10.04 g (175 ppm by mass in terms of the concentration of a phosphorus atom in a polyamide resin) of calcium hypophosphite ($Ca(H_2PO_2)_2$) and 7.26 g of sodium acetate which were accurately weighed, was sufficiently purged with nitrogen, and thereafter was filled with nitrogen until the inner pressure reached 0.4 MPa, and the mixture was heated to 190°C with the system being stirred under a small amount of nitrogen flow. The molar ratio of sodium acetate/calcium hypophosphite was 1.50.

Thereto was dropped 11,185 g (82.12 mol) of m-xylylenediamine with stirring, and the system was continuously heated with condensation water generated being removed outside the system. After the completion of dropping of m-xylylenediamine, the inner temperature was raised, the reaction container was placed under reduced pressure at the time of point where the inner temperature reached 265°C, the inner temperature was further raised, and a melting polycondensation reaction was continued at 270°C for 10 minutes. Thereafter, the system was pressurized with nitrogen, the resulting polymerized product was taken out from a strand die and pelletized to provide about 21 kg of a polyamide resin (A1) pellet, and the pellet was defined as an oxygen barrier resin composition. The relative viscosity of the polyamide resin (A1) was 1.95.

<Low surface tension liquid-tightness test>

To distilled water was added 0.1% by mass of a surfactant Tween 80 (Polysorbate 80 manufactured by Kanto Chemical Co., Inc.), thereby producing a low surface tensile liquid (low surface tension liquid). After the gasket was disposed at a location corresponding to the nominal volume in the syringe barrel, the syringe was filled with the low surface tension liquid from a tip thereof and the tip of the nozzle was sealed. The syringe barrel into which the low surface tension liquid was included in a sealed manner was left to still stand in a constant temperature room at 23°C for two weeks, with the barrel being horizontally located. After the standing, the presence of leakage from the gasket section of the syringe barrel was confirmed. The following Table shows the number of syringe barrel(s) where such leakage occurred, among three syringe barrels.

[Table 2]

| Items | Unit | Example 3 |
|---|---|---|
| Material (intermediate layer) | - | Polyamide |
| Material (skin layer) | - | COP |
| Shape | - | 1 mL Long |
| Slidability | (N) | 5.0 |
| Low surface tension liquid-tightness test | - | 0/3 |
| Arithmetic average roughness Ra of inner surface of syringe barrel | (nm) | 5.9 |

(continued)

| Items | Unit | Example 3 |
|---|---|---|
| Arithmetic average roughness Ra of surface of gasket | (nm) | 26 |

It was found from Table 2 that any case where an oxygen barrier resin composition including a polyamide in an intermediate layer was used was again excellent in slidability and low surface tension liquid-tightness.

[Examples 4 to 5 and Comparative Examples 3 to 4]

Each syringe barrel was produced in the same manner except that, in Example 2, the degree of grinding of the mold in contact with the inner surface of the syringe barrel was modulated and the arithmetic average roughness Ra of the inner surface of the syringe barrel was changed to each value represented in the following Table, and the syringe barrel was evaluated in the same manner except that the above low surface tension liquid-tightness test was performed instead of the liquid-tightness test-air-tightness test in Example 2. The evaluation results are shown in the following Table.

[Table 3]

| Items | Unit | Example 4 | Example 5 | Comparative Example 3 | Comparative Example 4 |
|---|---|---|---|---|---|
| Shape | - | 2.25 mL | 2.25 mL | 2.25 mL | 2.25 mL |
| Slidability | (N) | 8.5 | 7.3 | 10.2 | 8.9 |
| Low surface tension liquid-tightness test | - | 0/3 | 0/3 | 0/3 | 3/3 |
| Arithmetic average roughness Ra of inner surface of syringe barrel | (nm) | 4.2 | 12.1 | 2.0 | 30.8 |
| Arithmetic average roughness Ra of surface of gasket | (nm) | 66 | 66 | 66 | 66 |

It was found from Table 3 that any syringe barrel having a surface roughness of the inner surface, of less than 4 nm, in terms of the arithmetic average roughness Ra was inferior in slidability and any syringe barrel having a surface roughness of more than 25 nm was reduced in low surface tension liquid-tightness.

[Example 6]

The same manner was made except that a gasket where the average arithmetic roughness (Ra) of the surface of the sliding contact section was adjusted to a value represented in the following Table was used in Example 2, and a syringe was evaluated in the same manner except that the above low surface tension liquid-tightness test was performed instead of the liquid-tightness test-air-tightness test in Example 2. The evaluation results are shown in the following Table.

[Table 4]

| Items | Unit | Example 6 |
|---|---|---|
| Shape | - | 2.25 mL |
| Slidability | (N) | 9.5 |
| Low surface tension liquid-tightness test | - | 0/3 |
| Arithmetic average roughness Ra of inner surface of syringe barrel | (nm) | 5.9 |
| Arithmetic average roughness Ra of surface of gasket | (nm) | 30 |

It was found from Table 4 that any syringe barrel having an average arithmetic roughness (Ra) of the surface of the sliding contact section, of 30 nm, was also again excellent in slidability and liquid-tightness.

Reference Signs List

10 ... syringe barrel, 12 ... cylindrical main body, 12A to 12C ... inner surface, 14 ... injection section, 16 ... insertion section, 20 ... gasket, 22 ... wetted section, 24 ... sliding contact section, 26 ... protrusion section, 30 ... plunger, 40 ... cap, 100 ... syringe

**Claims**

1.  A syringe (100) comprising

    a syringe barrel (10) comprising a cylindrical main body (12) comprising an injection section (14) and an insertion section (16), wherein
    the cylindrical main body (12) comprising a layered structure, and
    a surface roughness of an inner surface of the cylindrical main body (12) is 4 nm to 25 nm in terms of arithmetic average roughness (Ra),
    the syringe comprising:

    a gasket (20) which comprises a wetted section (22) and a sliding contact section (24) in contact with an inner surface of the syringe barrel (10) and which is inserted through the insertion section (14) of the syringe barrel (10),
    a plunger (30) installed to the gasket (20), and
    a cap (40) installed to the injection section of the syringe barrel (10),
    **characterized in that**
    a surface roughness of the sliding contact section of the gasket is 25 nm to 70 nm in terms of arithmetic average roughness (Ra).

2.  The syringe (100) according to claim 1, wherein a resin layer forming the inner surface of the cylindrical main body (12) comprises a cycloolefin-based polymer.

3.  The syringe according to one of the preceding claims, wherein the gasket (20) comprises an inactive film layer on a surface of at least one of the wetted section (22) and the sliding contact section (24).

4.  The syringe according to claim 3, wherein the inactive film layer comprises a fluororesin.

5.  The syringe according to claim 4, wherein the fluororesin is polytetrafluoroethylene.

6.  The syringe according to one of the preceding claims, wherein a surface roughness of an inner surface of the cylindrical main body (12) is 5 nm to 10 nm in terms of arithmetic average roughness (Ra).

7.  The syringe according to one of the preceding claims, wherein the cylindrical main body (12) is formed so that at least the resin layer forms the inner surface.


**Patentansprüche**

1.  Spritze (100), umfassend

    einen Spritzenzylinder (10), umfassend einen zylindrischen Hauptkörper (12), der einen Injektionsabschnitt (14) und einen Einführabschnitt (16) umfasst, wobei
    der zylindrische Hauptkörper (12) eine Schichtstruktur umfasst, und
    wobei eine Oberflächenrauheit einer inneren Oberfläche des zylindrischen Hauptkörpers (12) 4 nm bis 25 nm, ausgedrückt als arithmetisches Mittel der Rauheit (Ra), beträgt.
    die Spritze umfasst:

    eine Dichtung (20), die einen benetzten Abschnitt (22) und einen Gleitkontaktabschnitt (24) in Kontakt mit einer Innenfläche des Spritzenzylinders (10) umfasst und die durch den Einführungsabschnitt (14) des Spritzenzylinders (10) eingeführt wird,

einem Kolben (30), der an der Dichtung (20) angebracht ist, und
eine Kappe (40), die auf dem Injektionsabschnitt des Spritzenzylinders (10) angebracht ist,
**dadurch gekennzeichnet, dass**
eine Oberflächenrauheit des Gleitkontaktabschnitts der Dichtung 25 nm bis 70 nm, ausgedrückt als arithmetisches Mittel der Rauheit (Ra), beträgt.

**2.** Spritze (100) nach Anspruch 1, wobei eine Harzschicht, die die Innenfläche des zylindrischen Hauptkörpers (12) bildet, ein Polymer auf Cycloolefinbasis umfasst.

**3.** Spritze nach einem der vorstehenden Ansprüche, wobei die Dichtung (20) eine inaktive Filmschicht auf einer Oberfläche des benetzten Abschnitts (22) und/oder des Gleitkontaktabschnitts (24) umfasst.

**4.** Spritze nach Anspruch 3, wobei die inaktive Filmschicht ein Fluorharz umfasst.

**5.** Spritze nach Anspruch 4, wobei das Fluorharz aus Polytetrafluorethylen besteht.

**6.** Spritze nach einem der vorstehenden Ansprüche, wobei eine Oberflächenrauheit einer Innenfläche des zylindrischen Hauptkörpers (12) 5 nm bis 10 nm, ausgedrückt als arithmetisches Mittel der Rauheit (Ra), beträgt.

**7.** Spritze nach einem der vorstehenden Ansprüche, wobei der zylindrische Hauptkörper (12) so ausgebildet ist, dass zumindest die Harzschicht die Innenfläche bildet.


**Revendications**

**1.** Seringue (100) comprenant

un cylindre de seringue (10) comprenant un corps principal cylindrique (12) comprenant une section d'injection (14) et une section d'insertion (16), dans laquelle
le corps principal cylindrique (12) comprend une structure stratifiée, et
une rugosité de surface d'une surface interne du corps principal cylindrique (12) est de 4 nm à 25 nm en termes de rugosité moyenne arithmétique (Ra),
la seringue comprenant :

un joint d'étanchéité (20) qui comprend une section mouillée (22) et une section de contact coulissant (24) en contact avec une surface interne du cylindre de seringue (10) et qui est inséré à travers la section d'insertion (14) du cylindre de seringue (10),
un piston (30) installé sur le joint d'étanchéité (20), et
un capuchon (40) installé sur la section d'injection du cylindre de seringue (10),
**caractérisée en ce que**
une rugosité de surface de la section de contact coulissant du joint d'étanchéité est de 25 nm à 70 nm en termes de rugosité moyenne arithmétique (Ra).

**2.** Seringue (100) selon la revendication 1, dans laquelle une couche de résine formant la surface interne du corps principal cylindrique (12) comprend un polymère à base de cyclooléfine.

**3.** Seringue selon l'une des revendications précédentes, dans laquelle le joint d'étanchéité (20) comprend une couche de film inactif sur une surface d'au moins l'une parmi la section mouillée (22) et la section de contact coulissant (24).

**4.** Seringue selon la revendication 3, dans laquelle la couche de film inactif comprend une résine fluorée.

**5.** Seringue selon la revendication 4, dans laquelle la résine fluorée est un polytétrafluoroéthylène.

**6.** Seringue selon l'une des revendications précédentes, dans laquelle une rugosité de surface d'une surface interne du corps principal cylindrique (12) est de 5 nm à 10 nm en termes de rugosité moyenne arithmétique (Ra).

**7.** Seringue selon l'une des revendications précédentes, dans laquelle le corps principal cylindrique (12) est formé de sorte qu'au moins la couche de résine forme la surface interne.

Fig.1

Fig.2

Fig.3

(3a)

(3b)

Fig.4

Fig.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016019619 A
- WO 2013071138 A1
- US 2016017490 A1
- JP 2006045528 A
- JP H10314305 A
- WO 2011143509 A1
- EP 2957310 A1
- EP 2796159 A1
- WO 2013077436 A
- WO 2017090556 A
- JP 5300939 A
- JP 5317411 A